# EUROPEAN PATENT APPLICATION

(11) **EP 3 951 696 A1**
(43) Date of publication of application: **09.02.2022**
(21) Application number: 20779208.6
(22) Date of filing: 24.03.2020
(51) Int. Cl.: G06Q 50/10, G06F 16/9035, G06F 16/907

(54) **INFORMATION PROCESSING DEVICE AND PROGRAM**

(30) Priority: 27.03.2019 JP 2019061622
(71) Applicant: Japan Tobacco Inc., Tokyo 105-8422 (JP)
(72) Inventor: KURISU, Toshiharu, Tokyo 105-8422 (JP); SAKUMA, Sayako, Tokyo 105-8422 (JP); OKAMURA, Yusuke, Tokyo 156-0042 (JP); HABASHIMA, Yoshiyuki, Tokyo 156-0042 (JP)
(74) Representative: Bandpay & Greuter
(86) International application number: PCT/JP2020/012833
(87) International publication number: WO 2020/196447

(57) **Abstract**

The present invention enables discovery of a new expression likely to be supported as a smell or taste expression by many users. An acquisition unit 11 acquires appearance history of expressions used by a plurality of users from a SNS server device 20 via a network 40. An extraction unit 12 extracts, for each user attribute, an appearing expression, the appearance frequency of which exceeds a threshold value on the basis of the appearance history acquired by the acquisition unit 11. The extracted appearing expression is referred to as a frequently appearing expression. A presentation unit 13 presents the frequently appearing expression extracted by the extraction unit 12 as an expression candidate meaning a smell to a user who uses an input device 30. A registration unit 14 registers the smell and the expression in association with each other when the frequency of selection, by the user who uses the input device 30, of the expression presented to the user as an expression meaning the smell satisfies a determined condition.

## Description

### TECHNICAL FIELD

The present invention relates to a technique for describing a smell or taste of an object.

### BACKGROUND

Smells are known to provide various benefits to humans by stimulating the limbic system, which has a role in controlling emotion, behavior and memory. For example, Patent Document 1 discloses a method in which a subject smells a smell is asked a question about memories associated with the smell, and a numerical value is assigned to the answer, and is recorded.

### PRIOR ART DOCUMENTS

### PATENT DOCUMENT

[Patent Document 1] JP-A-2016-180830

### SUMMARY

### PROBLEM TO BE SOLVED

In general, descriptions that are used to describe smells tend to be limited in scope and do not readily change; the same applies to descriptions that are used to describe taste.

It is thus an object of the present invention to provide a mechanism by which a new description can be found to describe smells or taste, and which is adopted by a large number of users.

### SOLUTION

To solve the problems, according to one aspect of the invention, there is provided an information processing device including: an extraction unit that extracts descriptions having an appearance frequency that exceeds a threshold based on appearance histories of descriptions used by plural users; a presentation unit that presents the extracted descriptions to plural users as candidate descriptions corresponding to a smell or taste; and a registration unit that registers the smell or the taste and the description in association with each other in a case that a condition is satisfied in which a frequency of selection of the presented descriptions as descriptions corresponding to the smell or taste is determined.

The extraction unit may extract the description for each attribute of the user.

The extraction unit may extract a description for which a frequency of appearance used by users having a certain attribute exceeds a first threshold and the frequency of appearance used by users having an attribute other than the certain attribute is less than a second threshold.

The extraction unit may extract the appearance frequency during a time period corresponding to an attribute of users.

The extraction unit may extract using a threshold corresponding to an attribute of a user as the threshold used when extracting the appearance frequency.

The presentation unit may present the extracted description to a user having the attribute when the description is extracted by the extraction unit.

The presentation unit presents the extracted description to a user having an attribute similar to the attribute when the description is extracted by the extraction unit.

The presentation unit may use a different presentation period or presentation frequency for each attribute of users to be presented as a presentation period or presentation frequency when presenting the extracted description.

According to another aspect of the invention, there is provided a program that causes a computer to implement: an extraction unit that extracts descriptions whose frequency of appearance exceeds a threshold based on a history of appearance of descriptions used by plural users; a presentation unit that presents the extracted descriptions to plural users as candidate descriptions meaning smell or taste; and a registration unit that registers the smell or taste and the description in association with each other when a condition for determining the frequency of selection of the presented descriptions meaning smell or taste is satisfied.

### EFFECT OF THE INVENTION

The present invention enables a description to be found that describes smells or taste, and which is adopted by a large number of users.

### BRIEF DESCRIPTION OF THE DRAWINGS

FIG. 1 is a block diagram illustrating an overall configuration of an information processing system according to an embodiment of the present invention.
FIG. 2 is a block diagram illustrating an example of a hardware configuration of an information processing device according to an embodiment of the present invention.
FIG. 3 is a block diagram illustrating an example of a functional configuration of an information processing device.
FIG. 4 is a diagram illustrating an example of the appearance of a description in a database stored in the information processing device.
FIG. 5 is a diagram illustrating an example of a frequent description database stored in the information processing device.
FIG. 6 is a diagram illustrating an example of a registration of a description in the database stored in the information processing device.
FIG. 7 is a flowchart illustrating an example of the operation of the information processing device.
FIG. 8 is a diagram illustrating an example of extraction of a frequently used description.

### DETAILED DESCRIPTION

### CONFIGURATION

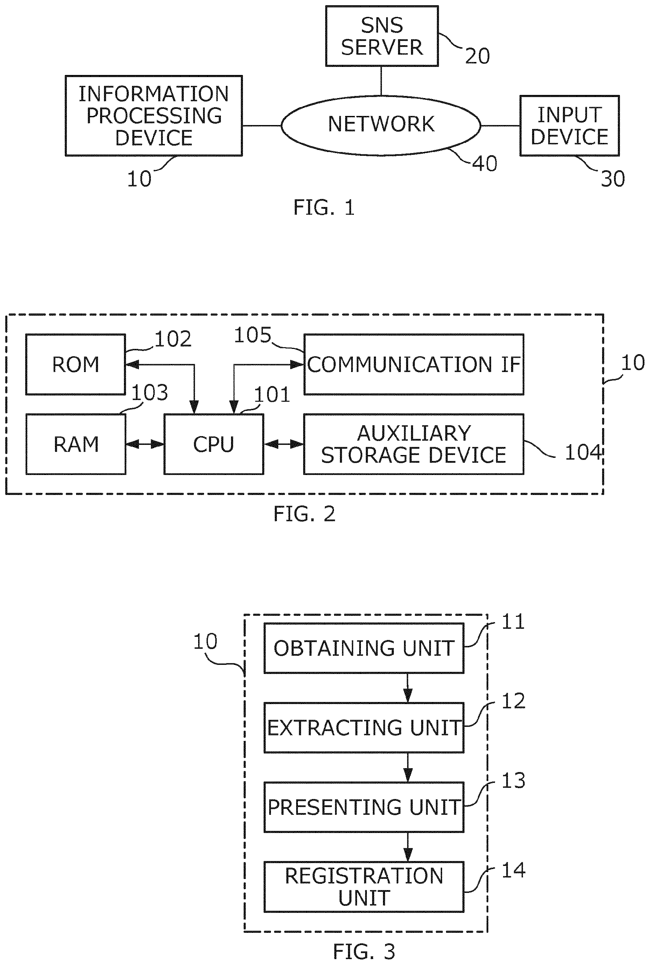

The overall configuration of an information processing system 1 according to an embodiment of the present invention will now be described. The information processing system 1 is a system for finding a new description for a smell of an object. The use of the new description is one that can be adopted by a large number of users, and which has not heretofore been commonly used for describing the smell of the object. As shown in FIG. 1, the information processing system 1 includes an information processing device 10, plural social networking service servers 20, and plural input devices 30. The information processing device 10, the SNS server 20, and the input device 30 communicate via a network 40 by either wired or wireless connection.

The information processing device 10 is an example of an information processing device according to the present invention, and is a device that controls other devices in the information providing system 1. The SNS server 20 is a device that stores information such as posts and comments from a large number of users and shares the information to devices that communicate with the network 40 in order to support communication between users. The information shared by the SNS server 20 includes various descriptions, for example, descriptions that are frequently used by people, descriptions that are prevalent, or descriptions that are rare. The input device 30 is provided in a showroom, an antenna shop, or the like to enable users to experience a smell of an object such as a perfume, smell oil, or herb, for example, and functions as a device for users to select a description that is considered suitable to describe the smell.

FIG. 2 is a diagram illustrating a hardware configuration of the information processing device 10. The information processing device 10 is a computer having a CPU (Central Processing Unit) 101, a ROM (Read Only Memory) 102, a RAM (Random Access Memory) 103, an auxiliary storage device 104, and a communication IF (Interface) 105. The CPU 101 is a processor that performs various operations. The ROM 102 is, for example, a non-volatile memory that stores a program and data used for booting the information processing device 10. The RAM 103 is a volatile memory that functions as a work area when CPU101 executes a program. The auxiliary storage device 104 is a nonvolatile storage device such as an HDD (Hard Disk Drive) or an SSD (Solid State Drive), and stores programs and data used in the information processing device 10. The CPU 101 executes the program to realize functions and operations that are described later. The communication IF 105 is an interface for performing communication in accordance with a predetermined communication standard. The communication standard may be a standard for either wired or wireless communication. In addition to the configuration illustrated in FIG. 2, the information processing device 10 may include other components such as a display device, consisting of a liquid crystal display or an operation device consisting of a keyboard.

FIG. 3 is a block diagram illustrating an example of the functional configuration of the information processing device 10. The information processing device 10 has functions of an obtaining unit 11, an extraction unit 12, a presentation unit 13 and a registration unit 14.

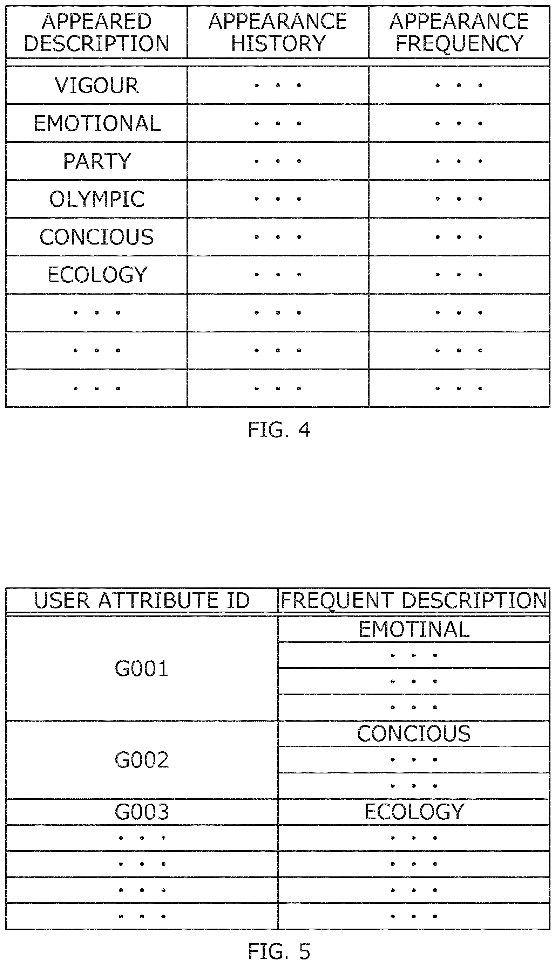

The obtaining unit 11 obtains an appearance history of a description that is used by a plurality of users from the SNS server 20 via the network 40. At this time, if the user attributes (for example, age, gender, region, occupation, hobby, etc.) of the user using the description can be obtained together with the description used, the obtaining unit 11 obtains the appearance history of the description together with the user attribute. Since the SNS server 20 may store the user attribute input by the user, the obtaining unit 11 obtains the user attribute from the SNS server 20 on condition that the user allows the attribute to be stored. In the auxiliary storage device 104, an appearance history database including an appearance history obtained by the obtaining unit 11 is stored (hereinafter, referred to as a DB). As shown in FIG. 4, the appearance history DB includes the appearance history of the description (hereinafter, referred to as "appearance description") appearing in the SNS server 20, and further includes the appearance frequency calculated based on the appearance history. Th appearance history and appearance frequency are classified according to the user attributes of the user using the appearance description. It is of note that when the appearance history and appearance frequency relate to users whose attributes are unknown, the attributes are categorized as user attribute unknown. In the example shown in FIG. 4, the appearance history and the appearance frequency of descriptions such as "vigor," "emotional," "party," "Olympic," "conscious," and "ecology" are classified according to user attributes.

The extraction unit 12 extracts an appearance description a frequency of which exceeds a threshold for each user attribute based on the appearance history obtained by the obtaining unit 11. The appearance descriptions extracted by the extraction unit 12 are referred to as frequent descriptions. The auxiliary storage device 104 stores a frequent description DB that includes frequent descriptions extracted by the extraction unit 12. As shown in FIG. 5, in the frequent description DB, frequent descriptions describe whose frequency of appearance exceeds a certain threshold for a first time within a certain period are recorded for each user attribute. In the example shown in FIG. 5, frequent descriptions such as "emotional" are extracted for the user group belonging to the user attribute to which the user attribute ID "G001" is allocated, frequent descriptions such as "conscious" are extracted for the user group belonging to the user attribute to which the user attribute ID "G002" is allocated, and frequent descriptions such as "ecology" are extracted for the user group belonging to the user attribute to which the user attribute ID "G003" is allocated.

The presentation unit 13 presents the frequent descriptions extracted by the extraction unit 12 to the user using the input device 30 as candidate descriptions corresponding to a smell. Individual users using the input device 30 may not be necessarily identical with the individual users using the SNS server 20 described above. If the user using the input device 30 smells the smell of an object such as a perfume, smell oil, or herb, for example, the presenting unit 13 presents the frequent descriptions extracted by the extraction unit 12 as candidate descriptions corresponding to the smell. The candidate descriptions are presented by, for example, displaying the frequent descriptions on the input device 30. At this time, the presenting unit 13 presents candidate descriptions to users who have user attributes identical to the user attribute for the classification when frequent descriptions are extracted. For example, the presentation unit 13 presents the frequent description "emotional" extracted for the user group belonging to the user attribute "women in their twenties" as the candidate description corresponding to the smell to the user group belonging to the user attribute "women in their twenties." The user operates the input device 30 to select, from among the frequent descriptions presented as candidate descriptions in the input device 30, descriptions that he/she accepts as appropriate for describing the smell.

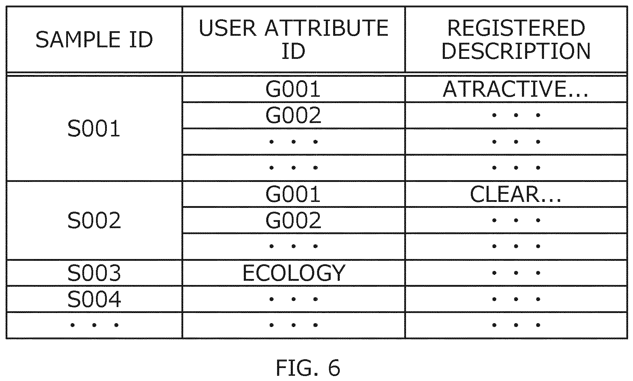

When the frequency, at which the frequent description presented to the user using the input device 30 is selected by the user as the description corresponding to the smell, satisfies a predetermined condition, the registration unit 14 registers the smell and the frequent description in association with each other. In short, for the frequency to satisfy the predetermined condition the presented frequent description has obtained a positive evaluation from a certain number of user groups as a description that corresponds to a particular smell. The registration description DB including the information registered by the registration unit 14 is stored in the auxiliary storage device 104. As shown in FIG. 6, in the registered description DB, the sample ID corresponding to the identifier of each smell and the frequent description whose frequency selected for the smell of the sample corresponding to the sample ID is equal to or higher than the threshold are associated with each other. The frequent description registered in the registration DB is referred to as a registered description. In FIG. 6, plural samples such as "attractive..." are registered as registered descriptions for the sample having the sample ID "ID001," and plural samples such as "clear..." are registered as registered descriptions for the sample having the sample ID "ID002." In the registered description DB, a general description that has been conventionally used as it corresponds to a smell is also registered in advance as a registered description. That is, in the registered description DB, in addition to a general description that has been conventionally used as a description corresponding to a smell, there is added a description that is newly registered by the registration unit 14 as a description corresponding to a smell.

### OPERATION

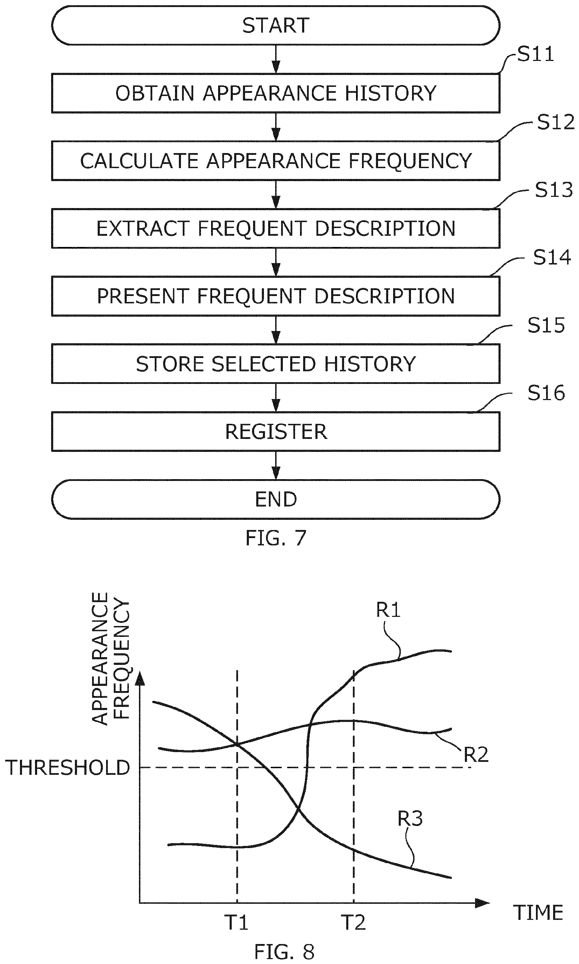

Operation of the present embodiment will be now be described with reference to the flowchart shown in FIG. 7. First, the obtaining unit 11 of the information processing device 10 obtains the appearance history for a large number of descriptions and the user attribute of the user using the appearance description from the SNS server 20, and registers (at step S11) the description and its appearance history in the appearance description DB (FIG. 4) for each user attribute.

Next, the obtaining unit 11 calculates the frequency of appearance for each user attribute based on the obtained appearance history, and registers it (at step S12) in the appearance description DB (FIG. 4) for each user attribute.

Next, based on the appearance frequency of the appearance description DB, the extraction unit 12 of the information processing device 10 extracts a frequent description whose appearance frequency exceeds a threshold for each user attribute, and registers it (at step S13) in the frequent description DB (FIG. 5). The appearance frequency exceeding the threshold is, for example, a case in which the number of appearances exceeds a certain threshold for the first time in the past month. More specifically, as shown in FIG. 7, the description corresponding to the number-of-appearances curve R1 in which the number of appearances per unit period (for example, one day) exceeds the threshold Th for the first time in the period T2-T1 (for example, one month) from the time T1 to the time T2 is extracted. On the other hand, the description corresponding to the number-of-appearances curve R2 in which the number of appearances per unit period has exceeded the threshold Th before the time T1, and the description corresponding to the number-of-appearances curve R3 in which the number of appearances per unit period has not exceeded the threshold Th in the period T2-T1 are not extracted. The trends in descriptions favored by the user may vary depending on the user attribute. Therefore, by this extraction processing, frequent descriptions that have started to become prevalent for each user attribute are extracted.

It is of note that a user who wishes to try various smells may visit a place where the input device 30 is installed, and inform the operator at the place that he/she wishes to try various smells. The user or operator manipulates the input device 30 to specify the sample ID of the sample desired by the user and enters the user attribute of the user. The presentation unit 13 of the information processing device 10 obtains the sample ID and the user attribute from the input device 30, searches the registration description group corresponding to the obtained sample ID and the frequent description group corresponding to the obtained user attribute in the frequent description DB in the registration description DB, and transmits the registration description group and the frequent description group obtained as a result of the search to the input device 30 as the candidate description corresponding to the smell, and presents (at step S14) the result to the user. That is, here, the registered description group already registered as the description of the smell of a certain sample and the frequent description group that has not yet been registered as the smell of the sample but has started to prevail in the user group having the same user attribute as the user are presented to the user as candidate descriptions corresponding to the smell. That is, the presentation unit 13 presents the description extracted by the extraction unit 12 to the user having the user attribute used by the extraction unit 12 to extract the description.

The user operates the input device 30 to select, from the registered description group and the frequent description group presented as candidate descriptions by the input device 30, a description that the user believes matches the smell of the smell. The registration unit 14 of the information processing device 10 obtains the selection result from the input device 30, and stores the sample ID of the smell of the sample corresponding to the selected description (registered description or frequent description), and the user attribute of the user in association with each other as a selection history (at step S15).

The registration unit 14 registers (at step S16) the sample ID of the sample, the frequent description, and the user attribute of the user as the registered description in the registered description DB in association with each other if the frequency of the frequent description selected as the description corresponding to the smell of the sample satisfies the determined condition, among the registered description group and the frequent description group presented to the user using the input device 30. A case where the frequency selected as the description indicating the smell of the sample satisfies the determined condition is, for example, a case where a number of times selected for a sample in the past one month by a user group having a certain user attribute exceeds a threshold. For example, when the number of times that the frequent description "emotional" in FIG. 5 is selected in the past month for the sample ID "S001" by the user group of the user attribute of the user attribute ID "G001" exceeds the threshold, it is newly registered as a description corresponding to the sample of the sample ID "S001" and the user attribute of the user attribute ID "G001" as shown in FIG. 6.

In this manner, a description that frequently appears is registered in the registered description DB upon being evaluated by a certain number of users as being suitable as a description of the smell. The contents of the registered description DB registered in this manner may be disclosed to the public as, for example, information on a smell, or may be used as reference information when an entity that manufactures a commodity such as a perfume or smell oil manufactures a new smell or engages in marketing.

According to the described embodiments it becomes possible to find a new description that is adopted by a large number of users as a description of a smell.

### MODIFICATION

The present invention is not limited to the embodiments described above. The embodiments described above may be modified as follows. Further, two or more items in the following modified examples may be combined.

### FIRST MODIFICATION

The present invention is applicable not only to a sense of smell but also to taste (e.g., gustatory objects such as wine, Japanese rice wine, spice, seasoning, etc.). That is, the present invention may be implemented by replacing "smell" with "taste."

### SECOND MODIFICATION

The extraction unit 12 may extract an appearance description in which the appearance frequency used by the user having a certain attribute exceeds the first threshold, and in which the appearance frequency used by the user having an attribute other than the certain attribute is less than the second threshold. According to this example, it is possible to extract an appearance description that is frequently used by a user group having a certain attribute (e.g., teenage women) and not frequently used by another user group having an attribute other than the stated attribute (e.g., women who are not teenagers); namely, a frequent description that is popularized only by a specific user group having a certain attribute. It is of note that either the first threshold or the second threshold may be larger than the other.

### THIRD MODIFICATION

The extraction unit 12 may perform extraction using a time period corresponding to a user attribute as a time period for extracting frequent descriptions. Specifically, the period T2-T1 illustrated in FIG. 7 may be shorter for teenage women who are considered to be sensitive to fashionable descriptions, such as [the time period for teenage women] < [the time period for women in their forties].

### FOURTH MODIFICATION

The extraction unit 12 may perform extraction using a threshold corresponding to a user attribute as a threshold used when extracting the frequency description specifically, the threshold Th exemplified in FIG. 7 may be increased for teenage women who are considered to be sensitive to fashionable descriptions, such as [Th for teenage women] > [Th for women in their forties].

### FIFTH MODIFICATION

In the embodiments, the presentation unit 13 presents the extracted frequent description to the user having the same user attribute as the user attribute when the frequent description is extracted by the extraction unit 12 (for example, if the former is a woman in her twenties, the latter is also a woman in her twenties). The presentation method of the frequent description is not limited thereto, and the presentation unit 13 may present the extracted frequent description to a user having a user attribute similar to the user attribute when the frequent description is extracted by the extraction unit 12 (if the former is a woman in her twenties, and the latter is a teenage woman or a woman in her thirties). A similarity of the user attributes may be defined in advance, or may be determined in accordance with a ratio at which the same frequent description is extracted. For example, if there are multiple user attributes a rate at which some frequent descriptions are extracted is equal to or greater than the threshold, the user attributes may be determined to be similar.

### SIXTH MODIFICATION

The presentation period in which the presentation unit 13 presents frequent descriptions to the user and the presentation frequency to be presented may be different for each user attribute. For example, for teenage women who are considered to be sensitive to fashionable descriptions, the presentation period may be short or the presentation frequency may be high. That is, the presentation unit 13 may use a different presentation period or presentation frequency for each attribute of the user to be presented as the presentation period or presentation frequency when presenting the extracted frequent description.

### SEVENTH MODIFICATION

The extraction condition of the frequent description and the registration condition of the registered description exemplified in the embodiments are not limited to the exemplification of the embodiments. In short, the extraction condition for the frequent description may be a condition corresponding to a high frequency of use as the description used by the user. The registration condition for the registered description may be a condition corresponding to obtaining a certain positive evaluation as a description indicating a certain smell in the frequent description presented to the user.

### EIGHTH MODIFICATION

The present invention may be provided as an information processing method that includes processing steps performed in the information processing device 10. The present invention may also be provided as a program executed in the information processing device 10. Such a program may be provided on a recording medium such as an optical disk, or may be downloaded to a computer via a network such as the Internet, to be installed.

While the present invention has been described in detail above, it will be apparent to those skilled in the art that the present invention is not limited to the above-described embodiments. The present invention may be practiced as modifications and variations within the scope of the invention as defined by the claims. Accordingly, the description provided herein is for illustrative purposes only and is not limitative of the invention.

### DESCRIPTION OF REFERENCE NUMERALS

1...Information processing system, 10...information processing device, 20...SNS server S device, 30...input device, 40...network, 101...CPU, 102...ROM, 103...RAM, 104...auxiliary storage device, 105...communication IF, 11...obtaining unit, 12...extraction unit, 13...presentation unit, 14...registration unit.

## Claims

1. An information processing device comprising:
an extraction unit that extracts descriptions having an appearance frequency that exceeds a threshold based on appearance histories of descriptions used by plural users;
a presentation unit that presents the extracted descriptions to plural users as candidate descriptions corresponding to a smell or taste; and
a registration unit that registers the smell or the taste and the description in association with each other in a case that a condition is satisfied in which a frequency of selection of the presented descriptions as descriptions corresponding to the smell or taste is determined.

2. The information processing device according to Claim 1, wherein
the extraction unit extracts the description for each attribute of the user.

3. The information processing device according to Claim 2, wherein
the extraction unit extracts a description for which a frequency of appearance used by users having a certain attribute exceeds a first threshold and the frequency of appearance used by users having an attribute other than the certain attribute is less than a second threshold.

4. The information processing device according to Claim 2 or 3, wherein
the extraction unit extracts the appearance frequency during a time period corresponding to an attribute of users.

5. The information processing device according to any one of Claims 2 to 4, wherein
the extraction unit extracts using a threshold corresponding to an attribute of a user as the threshold used when extracting the appearance frequency.

6. The information processing device according to any one of Claims 2 to 5, wherein
the presentation unit presents the extracted description to a user having the attribute when the description is extracted by the extraction unit.

7. The information processing device according to any one of Claims 2 to 6, wherein
the presentation unit presents the extracted description to a user having an attribute similar to the attribute when the description is extracted by the extraction unit.

8. The information processing device according to any one of Claims 2 to 7, wherein
the presentation unit uses a different presentation period or presentation frequency for each attribute of users to be presented as a presentation period or presentation frequency when presenting the extracted description.

9. A program that causes a computer to implement:
an extraction unit that extracts descriptions whose frequency of appearance exceeds a threshold based on a history of appearance of descriptions used by plural users;
a presentation unit that presents the extracted descriptions to plural users as candidate descriptions meaning smell or taste; and
a registration unit that registers the smell or taste and the description in association with each other when a condition for determining the frequency of selection of the presented descriptions meaning smell or taste is satisfied.
